(19) 

Europäisches
Patentamt
European
Patent Office
Office européen
des brevets

(11) **EP 3 903 885 A1**

(12) **EUROPEAN PATENT APPLICATION**
published in accordance with Art. 153(4) EPC

(43) Date of publication:
**03.11.2021 Bulletin 2021/44**

(21) Application number: **19902144.5**

(22) Date of filing: **24.12.2019**

(51) Int Cl.:
*A61P 43/00* (2006.01)     *A61P 17/00* (2006.01)
*A61K 36/185* (2006.01)     *A61K 36/28* (2006.01)
*A61K 36/31* (2006.01)     *A61K 36/48* (2006.01)
*A61K 36/736* (2006.01)     *A61K 36/76* (2006.01)
*A61K 36/882* (2006.01)

(86) International application number:
**PCT/JP2019/050479**

(87) International publication number:
**WO 2020/138023 (02.07.2020 Gazette 2020/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.12.2018 JP 2018245318**

(71) Applicant: **Suntory Holdings Limited
Osaka 530-8203 (JP)**

(72) Inventor: **AKAZAWA, Sota
Soraku-gun, Kyoto 619-0284 (JP)**

(74) Representative: **Vossius & Partner
Patentanwälte Rechtsanwälte mbB
Siebertstraße 3
81675 München (DE)**

(54) **AGENT FOR INHIBITING REDUCTION IN DECOMPOSITION OF DENATURED ELASTIN, AGENT FOR MAINTAINING NORMAL ELASTIN FIBERS, AGENT FOR INHIBITING FORMATION OF ELASTIN-ELAFINE COMPOSITE, AND SCREENING METHOD FOR SUBSTANCE HAVING ELASTIN-ELAFINE COMPOSITE FORMATION INHIBITORY EFFECT**

(57)    The present invention aims to provide an agent for inhibiting formation of an elastin-elafin complex and a composition for inhibiting formation of an elastin-elafin complex, which are capable of inhibiting formation of an elastin-elafin complex, a method of screening for a substance having an effect of inhibiting formation of an elastin-elafin complex, a method of inhibiting formation of an elastin-elafin complex, a method of inhibiting a reduction in breakdown of denatured elastin, a method of maintaining normal elastin fibers, and the like. The present invention relates to, for example, an agent for inhibiting a reduction in breakdown of denatured elastin, the agent containing, as an active ingredient, at least one plant extract selected from the group consisting of passion fruit extract, pot marigold extract, white mustard extract, marshmallow extract, white willow extract, calamus extract, peach kernel extract, and soybean extract.

**Description**

TECHNICAL FIELD

The present invention relates to an agent for inhibiting a reduction in breakdown of denatured elastin, an agent for maintaining normal elastin fibers, and an agent for inhibiting formation of an elastin-elafin complex. The present invention also relates to a composition for inhibiting a reduction in breakdown of denatured elastin, a composition for maintaining normal elastin fibers, and a composition for inhibiting formation of an elastin-elafin complex. The present invention still also relates to a method of screening for a substance having an effect of inhibiting formation of an elastin-elafin complex. Further, the present invention relates to a method of inhibiting formation of an elastin-elafin complex, a method of inhibiting a reduction in breakdown of denatured elastin, a method of maintaining normal elastin fibers, and the like. Still further, the present invention relates to use of a plant extract for inhibiting formation of an elastin-elafin complex, use of a plant extract for inhibiting a reduction in breakdown of denatured elastin, and use of a plant extract for maintaining normal elastin fibers.

BACKGROUND ART

Elastin is a major component of elastin fibers (elastic fibers) and is widely expressed in tissues such as skin, blood vessels, and lungs which require elasticity for expression of their functions. For example, in the dermis of the skin, elastin fibers function to impart elasticity to the skin and maintain resiliency, but the elastin fibers are degenerated by factors such as aging and ultraviolet light, resulting in loss of elasticity and the like. In particular, exposure of the skin to ultraviolet light over many years causes accumulation and deposition of denatured elastin on the dermis, which is considered to be a factor that causes scarce elasticity and many wrinkles, i.e., solar elastosis.

One of the factors that cause local accumulation of denatured elastin as observed in solar elastosis is considered to be reduced breakdown of denatured elastin.

In the dermis exposed to ultraviolet light, colocalization of elastin fibers and elafin is observed, which suggests formation of a protein complex between elastin and elafin. Elafin is an inhibitor of elastase. Elastin, once bonded to elafin to form a complex (elastin-elafin complex), shows resistance to elastase-mediated breakdown.

Meanwhile, it has been reported that some plant extracts have various useful effects. For example, Patent Literature 1 discloses a white mustard extract as a melanin production inhibitor.

CITATION LIST

- Patent Literature

Patent Literature 1: JP 2005-154375 A

SUMMARY OF INVENTION

- Technical Problem

As described above, an elastin-elafin complex formed by bonding between elastin and elafin shows resistance to elastase. Thus, formation of an elastin-elafin complex reduces breakdown of elastin and slows down elastin fiber turnover. Inhibiting formation of an elastin-elafin complex can, for example, inhibit a reduction in breakdown of denatured elastin and promote elastin fiber turnover. Inhibiting formation of an elastin-elafin complex is also expected to inhibit or reduce accumulation of denatured elastin so as to enable maintenance of normal elastin fibers. While Patent Literature 1 discloses a white mustard extract as a melanin production inhibitor, Patent Literature 1 is silent about studies on a substance having an effect of inhibiting formation of an elastin-elafin complex.

The present invention aims to provide an agent for inhibiting formation of an elastin-elafin complex and a composition for inhibiting formation of an elastin-elafin complex, which are capable of inhibiting formation of an elastin-elafin complex. The present invention also aims to provide a method of screening for a substance having an effect of inhibiting formation of an elastin-elafin complex. The present invention still also aims to provide a method of inhibiting formation of an elastin-elafin complex, a method of inhibiting a reduction in breakdown of denatured elastin, a method of maintaining normal elastin fibers, and the like.

- Solution to Problem

As a result of extensive studies to solve the above problem, the present inventor found that extracts of specific plants such as passion fruit have an effect of inhibiting formation of an elastin-elafin complex, and the present invention was thus completed.

The present invention encompasses but is not limited to the following agent for inhibiting formation of an elastin-elafin complex and the like.

(1) An agent for inhibiting a reduction in breakdown of denatured elastin, the agent containing, as an active ingredient, at least one plant extract selected from the group consisting of passion fruit extract, pot marigold extract, white mustard extract, marshmallow extract, white willow extract, calamus extract, peach kernel extract, and soybean extract.

(2) An agent for maintaining normal elastin fibers, the agent containing, as an active ingredient, at least one plant extract selected from the group consisting of passion fruit extract, pot marigold extract, white mustard extract, marshmallow extract, white willow extract, calamus extract, peach kernel extract, and soybean extract.

(3) The agent according to (1) or (2) above, wherein the agent inhibits formation of an elastin-elafin complex.

(4) An agent for inhibiting formation of an elastin-elafin complex, the agent containing, as an active ingredient, at least one plant extract selected from the group consisting of passion fruit extract, pot marigold extract, white mustard extract, marshmallow extract, white willow extract, calamus extract, peach kernel extract, and soybean extract.

(5) The agent for inhibiting formation of an elastin-elafin complex according to (4) above, wherein the agent is for use in promoting elastin fiber turnover by inhibiting formation of an elastin-elafin complex.

(6) The agent according to any one of (1) to (5) above, wherein the plant extract is at least one selected from the group consisting of passion fruit extract, pot marigold extract, and white mustard extract.

(7) A composition for inhibiting a reduction in breakdown of denatured elastin, the composition containing the agent for inhibiting a reduction in breakdown of denatured elastin according to (1), (3), or (6) above.

(8) A composition for maintaining normal elastin fibers, the composition containing the agent for maintaining normal elastin fibers according to (2), (3), or (6) above.

(9) A composition for inhibiting formation of an elastin-elafin complex, the composition containing the agent for inhibiting formation of an elastin-elafin complex according to any one of (4) to (6) above.

(10) The composition according to any one of (7) to (9) above, wherein the composition is a topical agent for skin.

(11) The composition according to any one of (7) to (10) above, wherein the composition is a cosmetic.

(12) The composition according to any one of (7) to (9) above, wherein the composition is a food or beverage.

(13) A method of screening for a substance having an effect of inhibiting formation of an elastin-elafin complex, the method including a step (a) of establishing contact between elafin and immobilized elastin in the presence or absence of a test substance, a step (b) of detecting whether or not bonding between the elafin and the immobilized elastin is inhibited by the test substance, and a step (c) of selecting the test substance as a substance having an effect of inhibiting formation of an elastin-elafin complex when the bonding between the elafin and the immobilized elastin is inhibited by the test substance.

(14) A method of inhibiting formation of an elastin-elafin complex, the method including administering at least one plant extract selected from the group consisting of passion fruit extract, pot marigold extract, white mustard extract, marshmallow extract, white willow extract, calamus extract, peach kernel extract, and soybean extract.

(15) A method of inhibiting a reduction in breakdown of denatured elastin, the method including administering at least one plant extract selected from the group consisting of passion fruit extract, pot marigold extract, white mustard extract, marshmallow extract, white willow extract, calamus extract, peach kernel extract, and soybean extract.

(16) A method of maintaining normal elastin fibers, the method including administering at least one plant extract selected from the group consisting of passion fruit extract, pot marigold extract, white mustard extract, marshmallow extract, white willow extract, calamus extract, peach kernel extract, and soybean extract.

(17) Use of a plant extract for inhibiting formation of an elastin-elafin complex, the plant extract being at least one selected from the group consisting of passion fruit extract, pot marigold extract, white mustard extract, marshmallow extract, white willow extract, calamus extract, peach kernel extract, and soybean extract.

(18) Use of a plant extract for inhibiting a reduction in breakdown of denatured elastin, the plant extract being at least one selected from the group consisting of passion fruit extract, pot marigold extract, white mustard extract, marshmallow extract, white willow extract, calamus extract, peach kernel extract, and soybean extract.

(19) Use of a plant extract for maintaining normal elastin fibers, the plant extract being at least one selected from the group consisting of passion fruit extract, pot marigold extract, white mustard extract, marshmallow extract, white willow extract, calamus extract, peach kernel extract, and soybean extract.

- Advantageous Effects of Invention

The present invention can provide, for example, an agent for inhibiting formation of an elastin-elafin complex and a composition for inhibiting formation of an elastin-elafin complex, which are capable of inhibiting formation of an elastin-elafin complex. The present invention can also provide a method of screening for a substance having an effect of inhibiting formation of an elastin-elafin complex.

Inhibiting formation of an elastin-elafin complex can inhibit a reduction in breakdown of denatured elastin. Inhibiting formation of an elastin-elafin complex can also contribute to maintaining normal elastin fibers. The present invention can provide a method of inhibiting formation of an elastin-elafin complex, a method of inhibiting a reduction in breakdown of denatured elastin, a method of maintaining normal elastin fibers, and the like.

DESCRIPTION OF EMBODIMENTS

The present invention is described below. The present invention is not limited to the following embodiments, and modifications can be made without departing from the scope of the present invention.

The agent for inhibiting formation of an elastin-elafin complex of the present invention contains, as an active ingredient, at least one plant extract selected from the group consisting of passion fruit extract, pot marigold extract, white mustard extract, marshmallow extract, white willow extract, calamus extract, peach kernel extract, and soybean extract.

As shown in Examples described later, the plant extracts each have an effect of inhibiting bonding between elastin and elafin and formation of a complex (elastin-elafin complex). The plant extracts are used to inhibit formation of an elastin-elafin complex. Inhibiting formation of an elastin-elafin complex can be rephrased as "inhibiting bonding between elastin and elafin".

As described above, formation of an elastin-elafin complex reduces breakdown of elastin. Inhibiting formation of an elastin-elafin complex can inhibit a reduction in breakdown of denatured elastin. Inhibiting a reduction in breakdown of denatured elastin is expected to promote elastin fiber turnover and inhibit or reduce accumulation of denatured elastin in tissues such as skin. Inhibiting a reduction in breakdown of denatured elastin is also expected to enable maintenance of normal elastin fibers.

In the present invention, the term "normal elastin fibers" refers to elastin fibers maintaining their inherent function and physical properties (e.g., strength and viscoelasticity). The term "denatured elastin" refers to elastin fibers and components thereof (e.g., tropoelastin and elastin breakdown products) whose function and physical properties are impaired as compared to those inherent thereto.

In the present invention, the simple term "elastin" and the term "elastin" in an elastin-elafin complex encompass tropoelastin, which is a precursor of elastin fibers, elastin in elastin fibers, and tropoelastin and/or elastin fibers in which partial structural change, breakdown, or fragmentation associated with aging or irritation are observed.

The expression "maintenance of normal elastin fibers" refers to inhibiting a reduction in the amount of normal elastin fibers and inhibiting a reduction in inherent function and physical properties. The phrase "inhibiting a reduction in the amount of normal elastin fibers and inhibiting a reduction in inherent function and physical properties" refers to inhibiting a reduction in the amount of normal elastin fibers and inhibiting a reduction in inherent function and physical properties when at least one of the plant extracts in the present invention is used as an active ingredient as compared to when none of the plant extracts in the present invention are used.

In one embodiment, the agent for inhibiting formation of an elastin-elafin complex of the present invention is suitably used to promote elastin fiber turnover by inhibiting formation of an elastin-elafin complex. The plant extracts each inhibit formation of an elastin-elafin complex, and can thus be used to inhibit a reduction in breakdown of denatured elastin and to maintain normal elastin fibers.

The present invention also encompasses an agent for inhibiting a reduction in breakdown of denatured elastin and an agent for maintaining normal elastin fibers, each of which containing, as an active ingredient, at least one plant extract selected from the group consisting of passion fruit extract, pot marigold extract, white mustard extract, marshmallow extract, white willow extract, calamus extract, peach kernel extract, and soybean extract.

Hereinafter, the agent for inhibiting formation of an elastin-elafin complex, the agent for inhibiting a reduction in breakdown of denatured elastin, and the agent for maintaining normal elastin fibers of the present invention are also collectively referred to as an "agent for inhibiting complex formation or the like".

In the agent for inhibiting complex formation or the like of the present invention, two or more of the plant extracts may be used in combination as active ingredients. In particular, at least one plant extract selected from the group consisting of passion fruit extract, pot marigold extract, and white mustard extract is preferred, because these extracts each have a higher effect of inhibiting formation of an elastin-elafin complex.

In the present invention, passion fruit is a fruit of *Passiflora edulis* (scientific name) belonging to the genus *Passiflora* of the family Passifloraceae. Pot marigold is *Calendula officinalis* (scientific name) belonging to the genus *Calendula* of the family Asteraceae. Pot marigold is also called calendula. White mustard is *Sinapis alba* (or *Brassica alba*) (scientific name) belonging to the genus *Sinapis* of the family Brassicaceae. Marshmallow is *Althaea officinalis* (scientific name) belonging to the genus *Althaea* of the family Malvaceae. Marshmallow is also called *Usubenitachioai*. White willow is *Salix alba* (scientific name) belonging to the genus *Salix* of the family Salicaceae. Calamus is *Acorus*

*calamus* (scientific name) belonging to the genus *Acorus* of the family Acoraceae Martinov. Peach kernel is a seed (persicae semen) of *Prunus persica* (or *Prunus persica* var. *davidiana*) (scientific name) belonging to the genes *Amygdalus* of the family Rosaceae. Soybean is *Glycine max* (scientific name) belonging to the genus *Glycine* of the family Fabaceae.

The passion fruit extract is *Passiflora edulis* fruit extract. The peach kernel extract is peach seed extract. The pot marigold, white mustard, marshmallow, white willow, calamus, and soybean extracts can be produced by extracting any portion (e.g., root, rhizome, stem, leaf, bark, flower, fruit, or seed) of these plants with a solvent. Two or more of these portions may be used in combination for extraction.

Preferably, the pot marigold extract is pot marigold flower extract. Preferably, the white mustard extract is white mustard seed extract. Preferably, the marshmallow extract is marshmallow root extract. Preferably, the white willow extract is white willow bark extract. Preferably, the calamus extract is calamus rhizome extract. Preferably, the soybean extract is soybean sprout extract.

Any plant extraction method may be used to obtain a plant extract. An extraction method commonly used to extract plant components can be used. The extraction method can be suitably selected, and extraction conditions are also not limited. For example, preferably, a plant extract is extracted from any of the above plants with a solvent (extraction solvent) at room temperature or under heating. In preparation of a plant extract, any of the above plants as raw materials may be directly extracted, or may be crushed, cut, or dried before extraction. In the present invention, a plant extract may be directly extracted from any of the above plants, or may be extracted from a crushed, cut, or dried product of any of the above plants. An extract from a crushed, cut, or dried product of any of the above plants is preferred.

The extraction solvent used to prepare a plant extract may be suitably selected, and one commonly used to extract plant components can be used. Examples of the extraction solvent include water; C1-C5 monohydric alcohols such as methanol, ethanol, propanol, and butanol; C2-C5 polyhydric alcohols such as ethylene glycol, propylene glycol, 1,2-butylene glycol, 1,3-butylene glycol, 1,4-butylene glycol, and 2,3-butylene glycol; ketones such as acetone and methyl ethyl ketone; esters such as methyl acetate and ethyl acetate; open-chain or cyclic ethers such as tetrahydrofuran and diethyl ether; polyethers such as polyethylene glycol; and squalane. Any of these may be used alone, or a mixed solvent (mixture) of two or more thereof may be used. Among the C1-C5 monohydric alcohols, C1-C4 monohydric alcohols are preferred; C2-C4 monohydric alcohols are more preferred; and ethanol is still more preferred. Among the C2-C5 polyhydric alcohols, C2-C4 di- or trihydric alcohols are preferred; dihydric alcohols are more preferred; and 1,3-butylene glycol is still more preferred. Of these, the extraction solvent is preferably water, a C1-C5 monohydric alcohol, a C2-C5 polyhydric alcohol, or a mixed solvent of two or more thereof; more preferably water, a C2-C4 monohydric alcohol or an aqueous solution thereof, or a C2-C4 dihydric alcohol or an aqueous solution thereof; still more preferably water, ethanol, an aqueous solution of ethanol, 1,3-butylene glycol, or an aqueous solution of 1,3-butylene glycol; particularly preferably water, an aqueous solution of ethanol, or an aqueous solution of 1,3-butylene glycol. In one embodiment, the concentration of ethanol or 1,3-butylene glycol in an aqueous solution of ethanol or an aqueous solution of 1,3-butylene glycol, respectively, is preferably 10 to 98 vol%, more preferably 30 to 90 vol%, still more preferably 30 to 70 vol%. The solvent extracts can be suitably used as the plant extracts in the present invention.

In one embodiment, preferably, the passion fruit extract, the pot marigold extract, and the marshmallow extract are extracted from the corresponding plants with an aqueous solution of 1,3-butylene glycol. Preferably, the white willow extract, the calamus extract, and the peach kernel extract are extracted from the corresponding plants with an aqueous solution of ethanol. Preferably, the white mustard extract and the soybean extract are extracted from the corresponding plants with water (preferably, hot water).

An acid or alkali may be added during extraction to adjust the pH of the extraction solvent. The extraction is preferably followed by removal of plant residues (the plant or its parts after extraction) from the resulting liquid extract. The method of removing the plant residues from the liquid extract is not limited. For example, a known separation means such as filtration or centrifugation can be used.

For example, the following method can be used as an example of the plant extraction method. A plant is directly finely crushed, or a dried product of a plant is finely crushed. Then, an extraction solvent in an amount 0.1 to 30 times that of the crushed product is added for extraction at normal pressure and room temperature preferably for 10 minutes to 15 days, more preferably 30 minutes to 10 days, still more preferably 1 hour to 7 days, or at a temperature near the boiling point of the extraction solvent preferably for about 10 minutes to 1 day (more preferably for 10 minutes to 2 hours), followed by filtration to obtain a filtrate. The extraction may be performed under standing still or suitable stirring. The resulting liquid filtrate (liquid plant extract) may be directly used as a plant extract, or may be diluted, concentrated, dried, or the like, if necessary.

In the present invention, a liquid plant extract obtained by extraction can be directly used as a plant extract. The liquid plant extract may be diluted, concentrated, or dried by a known method to provide a dilute solution, concentrate, or powder, or may be prepared in the form of a paste, as long as the effects of the present invention are not impaired. Examples of the drying method include freeze drying and spray drying. In addition, the liquid plant extract or its concentrate, dried powder, or the like may be further purified by deodorizing, decolorizing, or the like, if necessary, without impairing the effects of the present invention. Such a purification method may be any usual means.

The plant extracts in the present invention encompass liquid extracts extracted with various solvents which were obtained by the extraction method as described above, and diluted solutions, concentrates, and dried powders of such liquid extracts; and purified products thereof. The extracts may be diluted or dissolved in a solvent different from the extraction solvent.

The plant extracts are also commercially available, and commercial products can be used.

The agent for inhibiting complex formation or the like of the present invention may consist of one or more of the plant extracts, or may also contain other components and/or additives to be used in the form of a composition.

The agent for inhibiting formation of an elastin-elafin complex, the agent for inhibiting a reduction in breakdown of denatured elastin, and the agent for maintaining normal elastin fibers of the present invention can be directly used as an agent for inhibiting formation of an elastin-elafin complex, an agent for inhibiting a reduction in breakdown of denatured elastin, and an agent for maintaining normal elastin fibers, respectively. Each of these agents can also be added to a composition for inhibiting formation of an elastin-elafin complex or the like (described later). The agent for inhibiting complex formation or the like of the present invention is suitably used as an active ingredient of the composition for inhibiting formation of an elastin-elafin complex or the like.

A composition for inhibiting formation of an elastin-elafin complex, which contains the agent for inhibiting formation of an elastin-elafin complex of the present invention, is also encompassed by the present invention. A composition for inhibiting a reduction in breakdown of denatured elastin, which contains the agent for inhibiting a reduction in breakdown of denatured elastin of the present invention, is also encompassed by the present invention. A composition for maintaining normal elastin fibers, which contains the agent for maintaining normal elastin fibers of the present invention, is also encompassed by the present invention.

The composition for inhibiting formation of an elastin-elafin complex, the composition for inhibiting a reduction in breakdown of denatured elastin, and the composition for maintaining normal elastin fibers of the present invention are also collectively referred to as a "composition for inhibiting complex formation or the like". The composition for inhibiting complex formation or the like of the present invention contains at least one of the plant extracts as an active ingredient. The plant extracts and preferred embodiments thereof are as described above.

The agent for inhibiting complex formation or the like and the composition for inhibiting complex formation or the like of the present invention can be suitably used to inhibit formation of an elastin-elafin complex in the skin, to inhibit a reduction in breakdown of denatured elastin in the skin, or to maintain normal elastin fibers in the skin, for example.

The agent for inhibiting complex formation or the like and the composition for inhibiting complex formation or the like of the present invention are applicable for either therapeutic use (medical use) or non-therapeutic use (non-medical use).

The agent for inhibiting complex formation or the like and the composition for inhibiting complex formation or the like of the present invention, which contain at least one of the plants extract as an active ingredient, can be used to, for example, prevent or ameliorate conditions or symptoms for which it is desired to inhibit formation of an elastin-elafin complex. In one embodiment, the agent for inhibiting complex formation or the like and the composition for inhibiting complex formation or the like of the present invention can be used to, for example, prevent or ameliorate scarce elasticity or the like by inhibiting formation of an elastin-elafin complex. The agent for inhibiting complex formation or the like and the composition for inhibiting complex formation or the like of the present invention are also useful for cosmetic purposes such as maintenance of skin elasticity and prevention or amelioration of wrinkles and/or sagging by inhibiting formation of an elastin-elafin complex. The term "prevent" or "prevention" encompasses prevention of onset, delay of onset, and lower incidence. The term "ameliorate" or "amelioration" encompasses alleviation of symptoms, good reversal of symptoms, suppression of progression of symptoms, and cure of symptoms.

The composition for inhibiting complex formation or the like of the present invention can be used in various applications such as cosmetics, foods and beverages, and pharmaceutical and quasi-pharmaceutical products. The composition for inhibiting complex formation or the like of the present invention may be a cosmetic, a food or beverage, or a pharmaceutical or quasi-pharmaceutical product by itself for inhibiting formation of an elastin-elafin complex, for inhibiting a reduction in breakdown of denatured elastin, or for maintaining normal elastin fibers; or may be a material, a preparation, or the like that is added to such a cosmetic, food or beverage, pharmaceutical or quasi-pharmaceutical product, or the like.

The composition for inhibiting complex formation or the like of the present invention is suitably used as a topical agent for skin, for example. The topical agent for skin encompasses cosmetics and pharmaceutical and quasi-pharmaceutical products. Preferably, the topical agent for skin is a cosmetic. In one embodiment, in the case of using the composition for inhibiting complex formation or the like to inhibit formation of an elastin-elafin complex in the skin, to inhibit a reduction in breakdown of denatured elastin in the skin, or to maintain normal elastin fibers in the skin, the composition for inhibiting complex formation or the like is preferably provided as a topical agent for skin, and is more preferably provided as a cosmetic.

The composition for inhibiting complex formation or the like of the present invention can also be used as a pharmaceutical or quasi-pharmaceutical product, other than the topical agent for skin.

In another embodiment, the composition for inhibiting complex formation or the like can also be provided as a food or beverage. The following describes a case where the composition for inhibiting complex formation or the like of the present invention is provided as a topical agent for skin such as a cosmetic or a pharmaceutical or quasi-pharmaceutical product, a food or beverage, or the like.

When the composition for inhibiting complex formation or the like of the present invention is provided as a topical agent for skin, the dosage form and the like are not limited, and may be provided in any form. Examples include solutions, emulsions, creams, gels, powders, aerosols, mists, capsules, and sheets. Preferably, the topical agent for skin is a cosmetic. The product form of the cosmetic is also not limited. Examples include skin care cosmetics such as face wash, makeup remover, skin lotion, essence lotion, face pack, emulsion, cream, and sunscreen lotion; makeup cosmetics such as foundation, makeup base, lipstick, eyeshadow, eyeliner, mascara, eyebrow pencil, brush, and nail enamel; hair cosmetics such as shampoo, hair conditioner, hair styling products, hair dyes, and hair growth products; cleansing agents such as soap and body wash; and bath salts.

The above cosmetics may suitably contain one or more cosmetically acceptable carriers, additives, and like other components, without impairing the effects of the present invention. Examples include water, alcohols, oils, surfactants, thickeners, metal soaps, gelling agents, powders, chelating agents, water-soluble polymers, film forming agents, resins, inclusion compounds, antimicrobial agents, deodorants, salts, pH adjusting agents, ultraviolet light absorbers, extracts from microorganisms/plants/animals other than those mentioned above, keratolytic agents, enzymes, hormones, other vitamins, humectants, antiseptics, anti-inflammatory agents, and perfumes. The cosmetics can be produced by a usual production method in which, for example, any of the plant extracts is mixed with one or more cosmetically acceptable components described above, and the mixture is then processed into a desired form.

When the topical agent for skin is provided as a pharmaceutical or quasi-pharmaceutical product, components such as pharmaceutically or quasi-pharmaceutically acceptable carriers and additives may be used, without impairing the effects of the present invention. Examples of such components include excipients, binders, disintegrants, lubricants, antioxidants, and colorants. One or more of these components can be used, if necessary.

The composition for inhibiting complex formation or the like of the present invention can also be provided as a pharmaceutical or quasi-pharmaceutical product, other than the topical agent for skin described above. Such a pharmaceutical or quasi-pharmaceutical product may be administered orally or parenterally. The pharmaceutical or quasi-pharmaceutical product can be provided in the form of a formulation for oral administration (agent for internal use) or a formulation for parenteral administration. Examples of the dosage form of the formulation for oral administration include liquids, tablets, powders, fine granules, granules, sugar-coated tablets, capsules, suspensions, emulsions, and chewable tablets. Examples of the dosage form of the formulation for parenteral administration include injections and infusions. In such a pharmaceutical or quasi-pharmaceutical product, components such as pharmaceutically or quasi-pharmaceutically acceptable carriers and additives may be used. Examples of such components include excipients, binders, disintegrants, lubricants, antioxidants, colorants, and taste masking agents. One or more of these components can be used, if necessary. The pharmaceutical or quasi-pharmaceutical product can be produced by a usual production method in which, for example, any of the above plant extracts is mixed with one or more pharmaceutically or quasi-pharmaceutically acceptable components, and the mixture is then processed into a desired form.

When the composition for inhibiting complex formation or the like of the present invention is provided as a food or beverage, the food or beverage is not limited, and examples include general foods and beverages, health foods, foods with function claims, and foods for specified health uses. The form of the food or beverage is also not limited. The health foods, foods with function claims, and foods for specified health uses can be provided in the form of various formulations such as liquids, tablets, powders, fine granules, granules, sugar-coated tablets, capsules, suspensions, emulsions, chewable tablets, and liquid foods.

These foods and beverages may contain food- or beverage acceptable ingredients, such as other food or beverage materials and additives for foods and beverages, without impairing the effects of the present invention. Such food and beverages can be produced by a usual production method. For example, the method may only require adding any of the above plant extracts to food or beverage materials or the like in the production of foods and beverages.

When the composition for inhibiting complex formation or the like of the present invention is a topical agent for skin, a pharmaceutical or quasi-pharmaceutical product other than the topical agent for skin, or a food or beverage, the amount of the plant extract (in terms of dry matter) in the composition is preferably 0.0000001 to 100 wt%, more preferably 0.000001 to 100 wt%, still more preferably 0.00001 to 10 wt%, for example. The amount is the total amount when the composition contains two or more plant extracts.

The amount of the composition for inhibiting complex formation or the like of the present invention to be used is not limited, as long as the amount achieves the effect of inhibiting formation of an elastin-elafin complex, and can be suitably set according to the subject's conditions, weight, sex, age, or other factors. Preferably, the amount of the plant extract (in terms of dry matter) as a topical agent for skin such as a cosmetic is, for example, 0.01 to 500 mg per adult (60 kg) per day. Preferably, the dose of the plant extract (in terms of dry matter) when orally administered as a pharmaceutical or quasi-pharmaceutical product is, for example, 0.01 to 500 mg per adult (60 kg) per day. Preferably, the intake of the plant extract (in terms of dry matter) as a food or beverage is, for example, 0.01 to 500 mg per adult (60 kg) per day. The above amount of the plant extract can be applied, fed, or administered at once or in several portions. The timing at which a topical agent for skin containing the plant extract is applied (administered) to the skin and the timing at which a food, beverage, pharmaceutical product, or quasi-pharmaceutical product containing the plant extract is fed (administered) are not limited.

Subjects to which the composition for inhibiting complex formation or the like of the present invention is applicable (subject for administration) are not limited. For example, the composition is applicable to a human or non-human mammal, preferably a human. Examples of suitable subjects include those who want or need to inhibit formation of an elastin-elafin complex, those who want or need to inhibit a reduction in breakdown of denatured elastin, and those who want or need to maintain normal elastin fibers. Examples of such subjects include men and women who are concerned about undesirable changes in the skin, particularly wrinkles and/or sagging, due to ultraviolet light exposure (particularly, overexposure to ultraviolet light) and/or aging.

The present invention also encompasses the following uses and methods.

Use of a plant extract for inhibiting formation of an elastin-elafin complex, inhibiting a reduction in breakdown of denatured elastin, or maintaining normal elastin fibers, the plant extract being at least one selected from the group consisting of passion fruit extract, pot marigold extract, white mustard extract, marshmallow extract, white willow extract, calamus extract, peach kernel extract, and soybean extract.

A method of inhibiting formation of an elastin-elafin complex, the method including administering at least one plant extract selected from the group consisting of passion fruit extract, pot marigold extract, white mustard extract, marshmallow extract, white willow extract, calamus extract, peach kernel extract, and soybean extract; a method of inhibiting a reduction in breakdown of denatured elastin, the method including administering the plant extract; and a method of maintaining normal elastin fibers, the method including administering the plant extract.

Preferably, the plant extract is applied to the skin. The plant extract is suitably used to inhibit formation of an elastin-elafin complex in the skin; to inhibit a reduction in breakdown of denatured elastin in the skin; or to maintain normal elastin fibers in the skin.

In the uses and methods, the plant extracts, subjects for administration, preferred embodiments thereof, and the like are the same as described above for the agent for inhibiting complex formation or the like and the composition for inhibiting complex formation or the like of the present invention. Each plant extract may be directly used or may be used in combination with other components. The agent for inhibiting complex formation or the like or the composition for inhibiting complex formation or the like of the present invention may be used. Each plant extract can be used in the form of the topical agent for skin, food or beverage, or the like described above.

Each use may be therapeutic or non-therapeutic. Each method may be therapeutic or non-therapeutic. The "non-therapeutic" is a concept that does not include medical activities, i.e., a concept that does not include methods of surgery, therapy, or diagnosis of humans.

In other aspects, the present invention provides use of the at least one plant extract to produce an agent for inhibiting formation of an elastin-elafin complex or a composition for inhibiting formation of an elastin-elafin complex; use of the at least one plant extract to produce an agent for inhibiting a reduction in breakdown of denatured elastin or a composition for inhibiting a reduction in breakdown of denatured elastin; and use of the at least one plant extract to produce an agent for maintaining normal elastin fibers or a composition for maintaining normal elastin fibers. The plant extracts, preferred embodiments thereof, and the like are the same as described above for the agent for inhibiting complex formation or the like and the composition for inhibiting complex formation or the like of the present invention.

The present invention also encompasses a method of screening for a substance having an effect of inhibiting formation of an elastin-elafin complex. Next, the screening method of the present invention is described.

The method of screening for a substance having an effect of inhibiting formation of an elastin-elafin complex of the present invention includes a step (a) of establishing contact between elafin and immobilized elastin in the presence or absence of a test substance, a step (b) of detecting whether or not bonding between the elafin and the immobilized elastin is inhibited by the test substance, and a step (c) of selecting the test substance as a substance having an effect of inhibiting formation of an elastin-elafin complex when the bonding between the elafin and the immobilized elastin is inhibited by the test substance. The screening method of the present invention is an in vitro screening method.

The step (a) establishes contact between elafin and immobilized elastin in the presence or absence of a test substance.

Elastin immobilized to a solid-phase support in advance is used. Use of the immobilized elastin facilitates procedures such as washing.

Preferably, elastin to be immobilized is tropoelastin, $\alpha$-elastin, or the like. Particularly, elastin from human is preferred. Elastin from human or the like is commercially available, and a commercial product can be used. Elastin modified with a tag peptide may be used.

Any solid-phase support may be used. For example, a solid-phase support in the form of a microplate, microchip, glass slide, or the like can be used. The material of the solid-phase support is not limited. For example, a material such as plastic, glass, ceramic, metal oxide, or the like can be used.

For example, when the solid-phase support is a microplate such as a 96-well plate, an elastin solution obtained by dissolving or suspending elastin in a buffer is dispensed into each well of the microplate, and left to stand at 4°C to 37°C for 0.5 to 30 hours, whereby elastin can be immobilized. Any buffer may be used to prepare an elastin solution. A buffer such as a carbonate buffer or phosphate buffered saline (PBS) can be used. Preferably, the elastin concentration in the elastin solution is 1 to 100 μg/mL.

Preferably, the immobilized elastin and the solid-phase support are washed with a buffer such as PBS to remove unbound elastin molecules before the step (a). Preferably, after removal of unbound elastin molecules, a blocking solution (e.g., skim milk or PBS containing 1 to 10% albumin) is added for blocking.

Elafin from human is preferred. Elafin from human or the like is commercially available, and a commercial product can be used. Elafin is not limited as long as it binds to the immobilized elastin. Elafin modified with a tag peptide may be used. Elafin is not limited to a full-length protein. Elafin may be a protein that has an amino acid sequence with deletion, substitution, insertion, or addition of one or more (e.g., 1 to 9, 1 to 5, 1 to 3, 1 or 2, or 1) amino acid residues of a full-length elastin protein, and that can bind to elastin.

The test substance is not limited. Examples thereof include liquid plant extracts, cell extracts, supernatants of cell cultures, fermentation products, proteins, peptides, vitamins, and synthetic compounds. These may be known substances or novel substances.

The method of establishing contact between elafin and immobilized elastin in the presence of a test substance is not limited. For example, the contact can be established by incubating elafin and immobilized elastin in a solution containing a test substance. When performing the step (a) in the absence of a test substance, elafin and immobilized elastin may be incubated in a solution not containing a test substance. The solution can be a buffer such as PBS. The elafin concentration in the solution is not limited, but is preferably 0.1 to 10 μg/mL. For example, the test substance concentration may be suitably set to 1 to 100 μg/mL. Preferably, the pH (25°C) of the solution is 3 to 12. Serum or the like may be added to the solution to be used in the step (a). Preferably, contact between elafin and immobilized elastin is established at a temperature of 4°C to 40°C. The contact time between elafin and immobilized elastin may be usually 15 minutes to 24 hours, preferably 30 minutes to 2 hours.

After the step (a) and before the step (b), preferably, washing with buffer such as PBS is performed to remove unreacted molecules of the test substance and elafin.

The step (b) detects whether or not bonding between the elafin and the immobilized elastin is inhibited in the presence of the test substance. Whether or not the bonding is inhibited can be detected by quantifying the amount of elafin bonded to the immobilized elastin in the presence or absence of a test substance in the step (a), and comparing the amount of elafin bonded to the immobilized elastin in the presence of the test substance to the amount of elafin bonded to the immobilized elastin in the absence of the test substance.

The method of quantifying the amount of elafin bonded to the immobilized elastin is not limited. A method such as enzyme linked immunosorbent assay (ELISA) can be used. For example, an anti-elafin antibody is reacted with the immobilized elastin after the step (a), followed by washing to remove unreacted antibody. Subsequently, the anti-elafin antibody is reacted with a secondary antibody that reacts with the anti-elafin antibody, followed by washing to remove unreacted secondary antibody. The secondary antibody can be, for example, an antibody labeled with an enzyme such as horseradish Peroxidase (HRP) or alkaline phosphatase (AP) or a fluorescent dye, the antibody being capable of reacting with an anti-elafin antibody. A substrate corresponding to the enzyme such as HRP or AP bonded to the secondary antibody is allowed to react to develop color or emit light, or fluorescence is obtained by excitation. Then, the color development, light emission, or fluorescence is quantified using a plate reader or the like. Thus, the amount of elafin bonded to the immobilized elastin can be quantified.

In another embodiment, an anti-elafin antibody labeled with an enzyme such as HRP or AP, or a fluorescent dye is allowed to react with the immobilized elastin after the step (a), followed by washing to remove unreacted antibody. Subsequently, a substrate corresponding to the enzyme such as HRP or AP bonded to the antibody is allowed to react to develop color or emit light, or fluorescence is obtained by excitation. Then, the color development, light emission, or fluorescence is quantified using a plate reader or the like. Thus, the amount of elafin bonded to the immobilized elastin can be quantified.

The fluorescent dye can be Alexa Fluor® 488, Alexa Fluor® 596 (Thermo Fisher Scientific Inc.), or the like.

A comparison is made between the amount of elafin bonded to the immobilized elastin in the presence of the test substance and the amount of elafin bonded to the immobilized elastin in the absence of the test substance. When the amount of elafin bonded to the immobilized elastin is smaller in the presence than in the absence of the test substance, it is evaluated that the test substance inhibited the bonding between the elafin and the immobilized elastin.

In the step (b), when it is detected that the amount of elafin bonded to the immobilized elastin is smaller in the presence than in the absence of the test substance, it is determined that the presence of the test substance inhibited the bonding between the elafin and the immobilized elastin. The step (c) selects the test substance as a substance having an effect of inhibiting formation of an elastin-elafin complex, when the bonding between the elafin and the immobilized elastin is inhibited by the test substance.

The screening method of the present invention enables simple and efficient screening for a substance having an effect of inhibiting formation of an elastin-elafin complex.

EXAMPLES

The following describes the present invention with reference to examples, but the present invention is not limited to these examples.

<Example 1>

The following plant extracts were used as samples.

(1) Passion fruit extract: passion fruit (*Passiflora edulis* fruit) extract
(2) Pot marigold extract: pot marigold flower extract
(3) White mustard extract: white mustard seed extract
(4) Marshmallow extract: marshmallow root extract
(5) White willow extract: white willow bark extract
(6) Calamus extract: calamus rhizome extract
(7) Peach kernel extract: peach seed extract
(8) Soybean extract: soybean sprout extract

Each of the plant extracts was obtained by removing plant residues from a solvent extract (liquid extract) of the corresponding plant by filtration, concentrating the resulting filtrate in an evaporator, and drying the concentrate. For each of the plant extracts (1), (2) and (4) above, a crushed product of the plant was immersed in an aqueous solution of 1,3-butylene glycol in an amount 10 times the amount of the crushed product, and extracted over 10 minutes to 7 days with stirring once a day at room temperature. For each of the plant extracts (5), (6), and (7), the above method was used for extraction, except that a 30 to 90 vol% aqueous solution of ethanol was used instead of the aqueous solution of 1,3-butylene glycol.

For each of the plant extracts (3) and (8) above, the above method was used for extraction, except that hot water was used instead of the aqueous solution of 1,3-butylene glycol. The resulting powdered plant extracts were used for the following evaluation.

The samples were evaluated in terms of the effect of inhibiting formation of an elastin-elafin complex by solid phase binding assay. The solid phase binding assay was performed by the following method. A dilution buffer was a 0.5% serum albumin-containing phosphate buffered saline (PBS). Each plant extract (powder) was dissolved in dimethyl sulfoxide (DMSO).

A solution obtained by diluting tropoelastin and serum albumin to a concentration of 20 $\mu$g/mL in a bicarbonate buffer (14 mM sodium hydrogen carbonate, 6 mM sodium carbonate) was added in an amount of 100 $\mu$L to each well of a 96-well plate.

The 96-well plate was sealed with a parafilm, followed by reaction at 4°C for 24 hours. The reaction solution was removed, and a wash buffer (0.5% Tween 20 in PBS) was added in an amount of 150 $\mu$L to each well, followed by removal of the wash buffer. This procedure was repeated three times. Hereinafter, this step is referred to as "washing out". Then, a blocking buffer (5% skim milk in PBS) was added in an amount of 100 $\mu$L to each well, followed by reaction at room temperature for one hour. After washing out the blocking buffer, a complex formation reaction was performed. For the complex formation reaction of each sample, a complex reaction solution (a dilution buffer containing elafin (final concentration: 1 $\mu$g/mL), the plant extract (final concentration: 10 pg/mL), and DMSO (final concentration: 0.1%)) was added in an amount of 50 $\mu$L to each well for reaction at 37°C for one hour.

After washing out the complex reaction solution, an elafin-recognizing primary antibody reaction solution was added for reaction at 37°C for one hour. After washing out the primary antibody reaction solution, a horseradish peroxidase label-specific secondary antibody reaction solution was added for reaction at room temperature for one hour. After washing out the secondary antibody reaction solution, a TMB (3,3',5,5'-tetramethylbenzidine) solution was added in an amount of 100 $\mu$L to each well for reaction at room temperature for 30 minutes. 1 M phosphoric acid was added in an amount of 100 $\mu$L to each well to terminate the reaction. Then, the absorbance was measured using a microplate reader (wavelength: 450 nm).

As a negative control, a dilution buffer containing elafin (final concentration: 1 $\mu$g/mL) and DMSO (final concentration: 0.1%) was added in an amount of 50 $\mu$L to each well, instead of the complex reaction solution containing elafin and the plant extract, in the complex formation reaction. As a background, a dilution buffer containing DMSO (final concentration: 0.1%) (not containing either elafin or plant extracts) was added in an amount of 50 $\mu$L to each well, instead of the complex reaction solution containing elafin and the plant extract in the complex formation reaction. Other than that, the reaction with the antibody reaction solution was performed by the same method as in the samples containing the plant extracts, and the absorbance was measured using a microplate reader (wavelength: 450 nm).

Reagents and the like used in the assay were all commercial products. Tropoelastin was unmodified full-length human tropoelastin (#T0706 available from Sigma-Aldrich). Elafin was full-length human elafin to which a polyhistidine tag (His-tag) was added to the C-terminal (#12187-H08H available from Sino Biological Inc.).

The elastin-elafin complex formation inhibition rate (%) was calculated from the absorbance at 450 nm of each of the plant extracts (samples), the negative control, and the background, using the following calculation formula. The test was performed with n = 4 to 6 for each of the plant extracts, the negative control, and the background, and the complex formation inhibition rate was determined from the average absorbance at 450 nm.

```
Complex formation inhibition rate (%) = 100 - 100 × (Ab(S)
- Ab(NC))/(Ab(PC) - Ab(NC))
```

In the formula, Ab(S) is the absorbance at a wavelength of 450 nm of the plant extract (sample). Ab(NC) is the absorbance at a wavelength of 450 nm of the background. Ab(PC) is the absorbance at a wavelength of 450 nm of the negative control. Dunnett's test was used for the significant test (vs. negative control) (significance level: $p < 0.05$).

Table 1 shows the elastin-elafin complex formation inhibition rate of each plant extract. The complex formation inhibition rate of each of the passion fruit extract, the pot marigold extract, and the white mustard extract was found to be significant as compared to the negative control ($p < 0.05$).

[Table 1]

| Sample | Complex formation inhibition rate (%) |
|---|---|
| Passion fruit extract | 16 |
| Pot marigold extract | 14 |
| White mustard extract | 17 |
| Marshmallow extract | 8 |
| White willow extract | 3 |
| Calamus extract | 5 |
| Peach kernel extract | 15 |
| Soybean extract | 5 |

In the method described in Example 1, it is possible to screen for a substance having an effect of inhibiting formation of an elastin-elafin complex, using any test substance other than the plant extracts. A test substance selected as a substance having an effect of inhibiting formation of an elastin-elafin complex can be used as an active ingredient to inhibit a reduction in breakdown of denatured elastin, to maintain normal elastin fibers, or to inhibit formation of an elastin-elafin complex.

**Claims**

1. An agent for inhibiting a reduction in breakdown of denatured elastin, the agent comprising, as an active ingredient:
   at least one plant extract selected from the group consisting of passion fruit extract, pot marigold extract, white mustard extract, marshmallow extract, white willow extract, calamus extract, peach kernel extract, and soybean extract.

2. An agent for maintaining normal elastin fibers, the agent comprising, as an active ingredient:
   at least one plant extract selected from the group consisting of passion fruit extract, pot marigold extract, white mustard extract, marshmallow extract, white willow extract, calamus extract, peach kernel extract, and soybean extract.

3. The agent according to claim 1 or 2,
wherein the agent inhibits formation of an elastin-elafin complex.

4. An agent for inhibiting formation of an elastin-elafin complex, the agent comprising, as an active ingredient:
at least one plant extract selected from the group consisting of passion fruit extract, pot marigold extract, white mustard extract, marshmallow extract, white willow extract, calamus extract, peach kernel extract, and soybean extract.

5. The agent for inhibiting formation of an elastin-elafin complex according to claim 4,
wherein the agent is for use in promoting elastin fiber turnover by inhibiting formation of an elastin-elafin complex.

6. The agent according to any one of claims 1 to 5,
wherein the plant extract is at least one selected from the group consisting of passion fruit extract, pot marigold extract, and white mustard extract.

7. A composition for inhibiting a reduction in breakdown of denatured elastin, the composition comprising:
the agent for inhibiting a reduction in breakdown of denatured elastin according to claim 1, 3, or 6.

8. A composition for maintaining normal elastin fibers, the composition comprising:
the agent for maintaining normal elastin fibers according to claim 2, 3, or 6.

9. A composition for inhibiting formation of an elastin-elafin complex, the composition comprising:
the agent for inhibiting formation of an elastin-elafin complex according to any one of claims 4 to 6.

10. The composition according to any one of claims 7 to 9,
wherein the composition is a topical agent for skin.

11. The composition according to any one of claims 7 to 10,
wherein the composition is a cosmetic.

12. The composition according to any one of claims 7 to 9,
wherein the composition is a food or beverage.

13. A method of screening for a substance having an effect of inhibiting formation of an elastin-elafin complex, the method comprising:

a step (a) of establishing contact between elafin and immobilized elastin in the presence or absence of a test substance;
a step (b) of detecting whether or not bonding between the elafin and the immobilized elastin is inhibited by the test substance; and
a step (c) of selecting the test substance as a substance having an effect of inhibiting formation of an elastin-elafin complex when the bonding between the elafin and the immobilized elastin is inhibited by the test substance.

14. A method of inhibiting formation of an elastin-elafin complex, the method comprising:
administering at least one plant extract selected from the group consisting of passion fruit extract, pot marigold extract, white mustard extract, marshmallow extract, white willow extract, calamus extract, peach kernel extract, and soybean extract.

15. A method of inhibiting a reduction in breakdown of denatured elastin, the method comprising:
administering at least one plant extract selected from the group consisting of passion fruit extract, pot marigold extract, white mustard extract, marshmallow extract, white willow extract, calamus extract, peach kernel extract, and soybean extract.

16. A method of maintaining normal elastin fibers, the method comprising:
administering at least one plant extract selected from the group consisting of passion fruit extract, pot marigold extract, white mustard extract, marshmallow extract, white willow extract, calamus extract, peach kernel extract, and soybean extract.

**17.** Use of a plant extract for inhibiting formation of an elastin-elafin complex,
the plant extract being at least one selected from the group consisting of passion fruit extract, pot marigold extract, white mustard extract, marshmallow extract, white willow extract, calamus extract, peach kernel extract, and soybean extract.

**18.** Use of a plant extract for inhibiting a reduction in breakdown of denatured elastin,
the plant extract being at least one selected from the group consisting of passion fruit extract, pot marigold extract, white mustard extract, marshmallow extract, white willow extract, calamus extract, peach kernel extract, and soybean extract.

**19.** Use of a plant extract for maintaining normal elastin fibers,
the plant extract being at least one selected from the group consisting of passion fruit extract, pot marigold extract, white mustard extract, marshmallow extract, white willow extract, calamus extract, peach kernel extract, and soybean extract.

## INTERNATIONAL SEARCH REPORT

| | International application No. |
|---|---|
| | PCT/JP2019/050479 |

**A. CLASSIFICATION OF SUBJECT MATTER**
A61P 43/00(2006.01)i; A61P 17/00(2006.01)i; A61K 36/185(2006.01)i; A61K 36/28(2006.01)i; A61K 36/31(2006.01)i; A61K 36/48(2006.01)i; A61K 36/736(2006.01)i; A61K 36/76(2006.01)i; A61K 36/882(2006.01)i
FI:    A61K36/185;   A61K36/28;   A61K36/31;   A61K36/76;   A61K36/882; A61K36/736; A61K36/48; A61P43/00 111; A61P17/00
According to International Patent Classification (IPC) or to both national classification and IPC

**B. FIELDS SEARCHED**

Minimum documentation searched (classification system followed by classification symbols)
A61P43/00;   A61P17/00;   A61K36/185;   A61K36/28;   A61K36/31;   A61K36/48; A61K36/736; A61K36/76; A61K36/882

Documentation searched other than minimum documentation to the extent that such documents are included in the fields searched
Published examined utility model applications of Japan      1922–1996
Published unexamined utility model applications of Japan    1971–2020
Registered utility model specifications of Japan            1996–2020
Published registered utility model applications of Japan    1994–2020

Electronic data base consulted during the international search (name of data base and, where practicable, search terms used)
WPI; JSTPlus/JMEDPlus/JST7580 (JDreamIII) ;
CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2013-60404 A (ICHIMARU PHARCOS CO., LTD.) 04.04.2013 (2013-04-04) claim 4, tables 4, 5, paragraphs [0110], [0083] | 1-11, 14-19 |
| X | 川上晋平, パセノール TM の肌への作用, FOOD STYLE21, 2016, vol. 20, no. 3, pp. 58-60 in particular, fig. 6, page 59, column 3, non-official translation (KAWAKAMI, Shinpei, "Action of paseno-ru TM on skin") | 1-9, 12, 14-19 |
| A | JP 2015-17081 A (TOYO SHINYAKU CO., LTD.) 29.01.2015 (2015-01-29) paragraphs [0004], [0005] | 1-12, 14-19 |
| A | JP 2005-343887 A (KAO CORP.) 15.12.2005 (2005-12-15) paragraph [0002] | 1-12, 14-19 |

☒ Further documents are listed in the continuation of Box C.     ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 March 2020 (10.03.2020) | 24 March 2020 (24.03.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2019/050479 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2018/33227 A1 (ORTHOGEN AG) 22.02.2018 (2018-02-22) page 3, paragraph [0001], page 9, lines 32-34 | 1-12, 14-19 |
| X | MARTIN, M. et al., "Anti-aging properties of a passion fruit extract, targeted on wrinkle formation.", Journal of Investigative Dermatology, 2017, vol. 137, no. 5, suppl. 1, p. S93, 538 entire text | 2, 3, 6, 8, 10-12, 16, 19 |
| X | JP 2016-534982 A (LABORATOIRES EXPANSCIENCE) 10.11.2016 (2016-11- 10) paragraphs [0007], [0090], [0138]-[0140] | 2, 3, 6, 8, 10-12, 16, 19 |

Form PCT/ISA/210 (continuation of second sheet) (January 2015)

## INTERNATIONAL SEARCH REPORT

| International application No. |
| --- |
| PCT/JP2019/050479 |

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.  Consequently, this international search report is restricted to the invention first mentioned in the claims; it is covered by claims Nos.: Invention including a "passion fruit extract" in claims 1 to 12 and 14 to 19

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT
Information on patent family members

| International application No. |
| --- |
| PCT/JP2019/050479 |

| Patent Documents referred in the Report | Publication Date | Patent Family | Publication Date |
| --- | --- | --- | --- |
| JP 2013-60404 A | 04.04.2013 | (Family: none) | |
| JP 2015-17081 A | 29.01.2015 | (Family: none) | |
| JP 2005-343887 A | 15.12.2005 | US 2005/0265946 A1 paragraphs [0002] US 2007/0116659 A1 US 2010/0189709 A1 | |
| WO 2018/33227 A1 | 22.02.2018 | JP 2019-529357 A paragraphs [0007], [0038] EP 3352770 A1 CN 108348549 A KR 10-2019-0046793 A US 2019/0231816 A1 | |
| JP 2016-534982 A | 10.11.2016 | US 2016/0235794 A1 paragraphs [0006], [0144], [0145], [0200]-[0202] WO 2015/044254 A1 EP 3060228 A1 FR 3010906 A1 CA 2924382 A CN 105579050 A KR 10-2016-0058832 A | |

Form PCT/ISA/210 (patent family annex) (January 2015)

17

**INTERNATIONAL SEARCH REPORT**

| International application No. |
|---|
| PCT/JP2019/050479 |

<Continuation of Box No. III>

Document 1: JP 2013-60404 A (ICHIMARU PHARCOS CO., LTD.), 04.04.2013 (2013-04-04), in particular, claim 4, tables 4, 5, paragraph [0110] (Family: none)

Document 2: 川上晋平, パセノールTMの肌への作用, FOOD STYLE21, 2016, vol. 20, no. 3, pp. 58-60, in particular, fig. 6, non-official translation (KAWAKAMI, Shinpei, "Action of paseno-ru TM on skin")

Document 3: JP 2015-17081 A (TOYO SHINYAKU CO., LTD.), 29.01.2015 (2015-01-29) in particular, paragraphs [0004], [0005] (Family: none)

Document 4: JP 2005-343887 A (KAO CORP.) 15.12.2005 (2005-12-15), in particular, paragraph [0002] & US 2005/0265946 A1, in particular, paragraph [0002] & US 2007/0116659 A1 & US 2010/0189709 A1

Document 5: WO 2018/33227 A1 (ORTHOGEN AG) 22.02.2018 (2018-02-22), in particular, page 3, paragraph [0001], page 9, lines 32-34 & JP 2019-529357 A, in particular, paragraphs [0007], [0038] & EP 3352770 A1 & CN 108348549 A & KR 10-2019-0046793 A & US 2019/0231816 A1

Claims are classified into the following 9 inventions.

(Invention 1) Invention including a "passion fruit extract" in claims 1 to 12 and 14 to 19

Document 1 discloses a wrinkle-improving agent having a passion fruit extract as an active ingredient (claim 4, tables 4, 5, and paragraph [0110]). Document 2 discloses a skin sagging-improving agent having a passion fruit seed extract as an active ingredient (fig. 6).

In the invention in claim 1, with respect to the first option, an "inhibitor of degradation reduction of modified-elastin including a passion fruit extract as an active ingredient," considering that it is common technical knowledge that elastin in skin maintains elasticity of skin and reduction and modification of elastin cause skin aging, such as wrinkles (if necessary, see paragraphs [0004] and [0005] of document 3, paragraph [0002] of document 4, and paragraphs [0007] and [0038] of document 5), it is recognized that inhibiting degradation reduction of modified-elastin is namely maintaining elasticity of skin and the final purpose is maintaining elasticity of skin and thereby improving wrinkles and sagging as described in paragraph [0014] of the present description.

Therefore, an "inhibitor of degradation reduction of modified-elastin including a passion fruit extract as an active ingredient" in the invention in claim 1 cannot be considered to provide a novel usage in light of the description of document 1 and document 2 and lacks novelty, and thus does not have a special technical feature.

This also applies to the parts "including a passion fruit extract as an active ingredient" in claims 3, 6, 7, and 10 to 12 referring to claim 1.

Therefore, the parts "including a passion fruit extract as an active ingredient" in claims 1, 3, 6, 7, and 10 to 12, which have been evaluated with respect to whether or not a special technical feature is present, are classified as invention 1.

In addition, the parts "including a passion fruit extract as an active ingredient" in claims 2, 4, 5, 8, 9, and 14 to 19 are classified as invention 1.

Form PCT/ISA/210 (extra sheet) (January 2015)

**INTERNATIONAL SEARCH REPORT**

International application No.

PCT/JP2019/050479

(Invention 2) Invention including a "marigold extract" in claims 1 to 12 and 14 to 19

With respect to the invention including a "marigold extract" in claims 1 to 12 and 14 to 19, in regard to each plant extract set forth in claim 1 of the present application, neither a region of plant to be extracted nor an extraction method is specified, and what components are contained in each extract is unclear, and thus it is not recognized that common components are contained in the passion fruit extract and the marigold extract, and it is recognized to have the common technical feature of "including a plant extract." However, since said technical feature does not make a contribution over the prior art in light of the disclosure of document 1 and 2, this technical feature cannot be considered a special technical feature. Furthermore, there are no other identical or corresponding special technical features. Furthermore, the invention including a "marigold extract" in claims 1 to 12 and 14 to 19 is not substantially identical or equivalent to any of the claims classified as invention 1.

Therefore, the invention including a "marigold extract" in claims 1 to 12 and 14 to 19 cannot be classified as invention 1, and thus is classified as invention 2.

Like this, other plant extracts are classified as the following.

(Invention 3) Invention including a "white mustard extract" in claims 1 to 12 and 14 to 19

(Invention 4) Invention including an "althea extract" in claims 1 to 12 and 14 to 19

(Invention 5) Invention including a "Salix alba extract" in claims 1 to 12 and 14 to 19

(Invention 6) Invention including an "Acorus calamus extract" in claims 1 to 12 and 14 to 19

(Invention 7) Invention including a "peach pit extract" in claims 1 to 12 and 14 to 19

(Invention 8) Invention including a "Glycine max extract" in claims 1 to 12 and 14 to 19

(Invention 9) Invention in claim 13

The invention in claim 13 cannot be considered to have a technical feature identical or corresponding to that of claims 1 to 12 and 14 to 19 classified as invention 1. In addition, claim 13 is not dependent on any of claims 1 to 12 and 14 to 19. Further, claim 13 is not substantially identical or equivalent to any of the claims classified as invention 1.

Thus, claim 13 is classified as invention 9.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005154375 A